Europäisches Patentamt

European Patent Office

Office européen des brevets

(19)

(11) Publication number: **0 603 393 A1**

(12) **EUROPEAN PATENT APPLICATION**
**published in accordance with Art.**
**158(3) EPC**

(21) Application number: **92914381.6**

(22) Date of filing: **06.07.92**

(86) International application number:
**PCT/JP92/00849**

(87) International publication number:
**WO 93/01214 (21.01.93 93/03)**

(51) Int. Cl.5: **C07K 15/14**, C12P 21/02,
//C12N15/24,(C12P21/02,
C12R1:91)

(30) Priority: **08.07.91 JP 167095/91**

(43) Date of publication of application:
**29.06.94 Bulletin 94/26**

(84) Designated Contracting States:
**AT BE CH DE DK ES FR GB GR IT LI LU MC
NL SE**

(71) Applicant: **Chugai Seiyaku Kabushiki Kaisha**
**5-1, 5-chome, Ukima**
**Kita-ku**
**Tokyo 115(JP)**

(72) Inventor: **HASEGAWA, Masakazu, Chugai**
**Seiyaku K K**
**135, Komakado 1-chome**
**Gotenba-shi**
**Shizuoka 412 uoka 412(JP)**
Inventor: **ORITA, Tetsuro, Chugai Seiyaku**
**Kabushiki Kaisha**
**135, Komakado 1-chome**
**Gotenba-shi**
**Shizuoka 412(JP)**
Inventor: **OCHI, Norimichi, Chugai Seiyaku**
**Kabushiki Kaisha**
**135, Komakado 1-chome**
**Gotenba-shi**
**Shizuoka 412(JP)**

(74) Representative: **Davies, Jonathan Mark**
**Reddie & Grose**
**16 Theobalds Road**
**London WC1X 8PL (GB)**

(54) RECOMBINANT HUMAN INTERLEUKIN 6 WITH HOMOGENEOUS N-TERMINUS AND PRODUCTION
THEREOF.

(57) A recombinant human interleukin 6 with a homogeneous N-terminus, comprising a peptide molecular species wherein the N-terminus is Val and which is modified with a saccharide chain; and a process for producing the interleukin 6 which comprises culturing animal cells transformed by the DNA which codes for a fusion protein composed of a human interleukin 6 wherein the N-terminus is Val and a signal peptide other than the one intrinsic to a human interleukin 6 and collecting the interleukin 6 from the medium.

Rank Xerox (UK) Business Services
(3.10/3.09/3.3.4)

# Fig.3

(KD)

92.5 ——

66.2 ——

45.0 ——

31.0 ——

21.5 ——

1 2 3 4 5

1. MOLECULAR WEIGHT MARKER
2. rhIL-6 (CHO)
3. NEURAMIDASE AND ENDO-α-N-ACETYLGALACTORAMINIDASE
4. NEURAMIDASE, ENDO-α-N-ACETYLGALACTORAMINIDASE AND GLYCOPEPTIDASE F
5. rhIL-6 (E. COLI)

FIELD OF THE ART

The present invention relates to recombinant human interleukin-6 comprising a homogeneous molecule species whose N-terminus starts with valine (Val) and which is glycosylated, and a process for production thereof.

BACKGROUND ART

Interleukin-6 is a physiologically active substance which is promising as pharmaceuticals especially as a thrombopoietic factor due to its biological activities such as support of the growth of hemopoietic cells. Interleukin-6 obtained as a native form by culturing human cells is a glycoprotein having sugar chains, and includes several species different in their N-terminus as shown in Table 1.

A process for production of interleukin-6 has been developed wherein an interleukin-6 gene is cloned by a recombinant DNA technology, and is introduced into animal cells such as Chinese hamster ovary cells, and the cells are cultured. Although purified interleukin-6 is modified with sugar chains similarly to the native type, its N-terminus was heterologous.

## Table 1

| Amino acid sequence of N-terminus | Source | Reference |
|---|---|---|
| A-P-V-P-P-G-E-(45%)<br>V-P-P-G-E-(55%) | MG-63<br>Human osteosarcoma | Eur, j. Biochem,<br>168, 543 (1987) |
| A-P-V-P-P-G-E-(++)<br>P-V-P-P-G-E-( +)<br>V-P-P-G-E-(++) | Human lucocyte | J. Immunol.<br>140, 1534 (1988) |
| A-P-V-P-P-G-E-( +)<br>P-V-P-P-G-E-( +)<br>V-P-P-G-E-(++)<br>P-P-G-E-( +)<br>P-G-E-( +)<br>G-E-(++) | Recombinant type<br>Host: CHO | Agric, Biol,<br>Chem,<br>54, 2685 (1990) |
| A-P-V-P-P-G-E-(++)<br>P-V-P-P-G-E-( +)<br>V-P-P-G-E-(++)<br>P-P-G-E-( +)<br>P-G-E-( +)<br>G-E-( +) | Recombinant type<br>Host: NIH3T3 | |

This heterogeneity of the N-terminus disturbs a purity assay of a purified protein. In addition, separation of each component is difficult, and assessments of safety, pharmacological effects, absorption, metabolism, excretion and the like were impossible. In addition, because of these reasons, the use of human interleukin-6 as pharmaceuticals was essentially impossible.

Note that although the production of recombinant human interleukin-6 having the homogeneous N-terminus using a microorganism such as E. coli is possible (Tonouchi, H. et al., J. Biochem. 104, 30 (1988)-), there are problems relating to antigenicity and the like because the polypeptide moiety otherwise covered with sugar chains is exposed resulting in the production of antibodies.

DISCLOSURE OF THE INVENTION

Accordingly, the present invention provides human interleukin-6 having the homogeneous N-terminus and a process for production thereof.

The present inventors, as a result of various research relating to homogenization of the N-terminus of interleukin-6, found that interleukin-6 which is produced by Chinese hamster ovary cells, has the homogeneous N-terminus and is modified with sugar chains by using a signal peptide of human granulocyte colony-stimulating factor and selecting Val as T-terminus of interleukin-6, and completed the present invention. There are advantages wherein interleukin-6 having the homogeneous N-terminus and modified with sugar chains is obtained without changing a conventional culturing and purification system.

Accordingly, the present invention provides recombinant interleukin-6 comprising a peptide molecule species whose N-terminus starts with Val and which has sugar chains, and having the homogeneous N-terminus.

The present invention further provides a process for production of human interleukin-6, characterized by culturing animal cells transformed with a DNA coding for a fusion protein comprising interleukin-6 whose T-terminus starts with Val and a signal peptide other than the native signal peptide of human interleukin-6, and recovering interleukin-6 from the resulting culture.

BRIEF EXPLANATION OF DRAWINGS

Figure 1 shows a process for construction of an expression plasmid for native interleukin-6.

Fig. 2 shows an expression plasmid prIL-6GA for human IL-6 whose N-terminus is Ala, and a plasmid prIL-6GV for human IL-6 of the present invention wherein the T-terminus is Val.

Fig. 3 is an electrophoretic view showing that human IL-6 of the present invention has sugar chains.

DETAILED DESCRIPTION

Next, the present invention is explained in detail.

The present invention provides a process for obtaining recombinant human interleukin-6 (hereinafter abbreviated as human IL-6) having the homogeneous N-terminus, by replacing a signal peptide and using animal cells as a host, and the recombinant human IL-6 produced by this process.

The signal peptide includes, for example, that of human granulocyte colony-stimulating factor (hereinafter, abbreviated as human G-CSF). The signal peptide of human IL-6 is rich in Pro which is statistically rarely found in signal peptides, and in the signal peptide of human IL-6 the distance between the hydrophobic core believed to be embedded in the cell membrane and the cleavage site is short. The signal peptide of human G-CSF is characterized in that it does not contain Pro near to the cleavage site, has an adequate distance between the hydrohobic core and the cleavage site, and repulses against the phospholipid bilayer because the signal peptide contains Glu having negative charge at -2 position, and therefore a portion near to the cleavage site is reliably exposed out of the cell membrane.

Accordingly, in comparison with human IL-6 having the above-mentioned properties, signal peptides having the properties like the signal peptide of the human G-CSF can be used in the present invention. Such signal peptides include, for example, signal peptides of mouse IL-6, mouse granulocyte colony stimulation factor, human erythropoietin, mouse erythropoietin, human granulocyte macrophage colony-stimulating factor, mouse granulocyte macrophage colony-stimulating factor, human IL-2, mouse IL-2, human IL-3, mouse IL-3, human IL-4, mouse IL-4, human IL-5, mouse IL-5, human IL-7, mouse IL-7, human interferon (IFN)$\alpha$, mouse IFN$\beta$, human IFN$\gamma$, mouse IFN$\gamma$, human macrophage colony-stimulating factor, mouse macrophage colony-stimulating factor, and the like.

In addition, signal peptides may be derived from the native signal peptide of human IL-6 by modification at its C-terminal part.

Animal cells as host include Chinese hamster ovary cells (hereinafter abbreviated as CHO) and the like. CHO cells have been most frequently used, for example, for the production of recombinant human G-CSF, and the structure of sugar chains added by this cell line is most similar to that of the native type. However, animal cells as host are not limited to CHO, and for example, various cells lines such as NIH3T3, C127, BHK, Namalva, etc., can be used.

As the N-terminal sequence of human IL-6, Val-Pro-Pro-Gly-Glu-Asp- is chosen. Where Ala-Pro-Val-Pro-Pro-Gly- was chosen as the N-terminal sequence by merely replacing the signal peptide, a homogeneous N-terminus was not able to be obtained; and only where Val-Pro-Pro-Gly-Glu-Asp- was chosen, was the homogeneous N-terminus obtained. It is obvious that the above-fact cannot be easily conceived from the

4

structure of human IL-6. In addition, as this sequence is found in native type sequences, there is no fear of antigenicity as found in artificially modified sequences.

According to the present invention, human IL-6 produced by a host is mainly extracellularly secreted. Accordingly, recombinant human IL-6 of the present invention can be obtained from a culture supernatant after culturing, and applying conventional purification means such as ammonium sulfate fractionation, ethanol fractionation, acetone fractionation, gel filtration chromatography, reverse chromatography, anion exchange chromatography, hydrophobic chromatography, preparative electrophoresis, affinity chromatography, etc.

EXAMPLES

Next, Examples of the present invention are shown. In the cases where not specifically described, gene manupuration was carried out according to Molecular Cloning, Cold Spring Harbor Laboratory (1989), and enzymes were purchased from Takara Shuzo. Oligonucleotides were synthesized by an automatic DNA synthesizer (Applied Biosystems), and DNAs were prepared by low melting agarose electrophoresis.

Reference Example 1. Expression of recombinant human IL-6

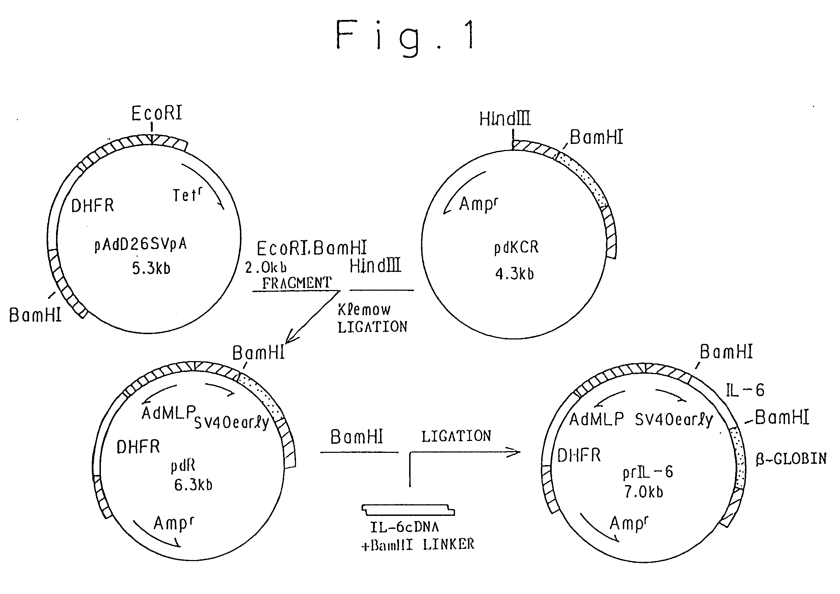

A 2.0 kb EcoRI/BamHI fragment containing a dihydrofolate reductase (abbreviated as DHFR hereinafter) of plasmid pAdD26SVpA (Kaufman, R.J., and Sharp, P.A., Mol. Cell. Biol., 2, 1304 (1982)) was blunt-ended by Klenow enzyme. Similarly, Hind-III site of plasmid pdKCRO' (Fukunaga, R. et al., Proc. Natl. Acad. Sci. USA, 81, 5086 (1984)) was blunt-ended, and this plasmid was ligated with the above-mentioned fragment to construct plasmid pdR (see, Fig. 1).

A DNA fragment comprising a portion from 19 position to 678 position of human IL-6 gene described in Hirano, T. et al., Nature, 324, 73 (1986) and further having BamHI linker at both the ends was inserted into the BamHI site under the control of an SV40 early promoter of plasmid pdR to obtain plasmid prIL-6 (see Fig. 1).

Cells of a CHO DXB-11 cell line which is a DHFR gene-deletion mutant (Graf, L.H. and Chasin, L.A., Mol. Cell Biol., 2, 93 (1982)) were transformed with the above-mentioned plasmid prIL-6 according to a calcium phosphate method.

DHFR positive cells were selected on nucleic acid-free Alpha MEM (GIBCO), and selection was repeated in a medium supplemented with methotrexate (abbreviated as MTX hereinafter) which is a folic acid analog to amplify the incorporated gene. A clone resistant to 100 nM or 50 nM MTX was clutured in Iskov's modified D'MEM (GIBCO) containing 1% fetal bovine rerum for three days, and the culture supernatant was collected as a starting material.

Purification was carried out by using gel filtration and reverse-phase chromatography. Namely, the culture supernatant was concentrated by about 8 times original concentration by ultrafiltration using Amicon PM-10 membrane (Amicon), and added to TSK-G3000SW column (21.5 mm × 60 cm, Toso) equilibrated with 50 mM Tris-HCl buffer (pH7.4) containing 150 mM NaCl and 0.05% Tween 20, and development was carried out in the same buffer at a flow rate of 3.75 mℓ/minute.

As a result of detection of the resulting fractions with anti-IL-6 antibody (Genzyme), IL-6 was eluted at a duration time of 42 to 50 minutes. The fractions containing IL-6 were collected, again concentrated with PM-10 membrane, and subjected to reverse-phase HPLC. Namely, the sample was injected into a Vydac protein C4 column (Vidac)(4.6 mm × 25 cm) equilibrated with 35% acetonitrile containing 0.1% trifluoroacetic acid, and adsorbed proteins were eluted by increasing the acetonitrile concentration from 35% to 80% in a linear gradient of 0.5 %/min. at a flow rate of 1 mℓ/minute.

As a result of detection of the resulting fractions with the above-mentioned antibody, IL-6 was eluted at an acetonitrile concentration of 43 to 48%. A fraction containing IL-6 was diluted 2 fold by addition of distilled water, and again subjected to reverse-phase HPLC under the same condition to obtain purified IL-6.

The N-terminal amino acid sequence of IL-6 was analyzed by an online system of a gas phase protein sequencer 470A type and PTH analyzer 120 type (Applied Biosystems).

As a result, the N-terminal amino acid sequences of recombinant human IL-6 were mixture of Ala-Pro-Val-Pro-Pro-Gly- which was the same as the native type, and Val-Pro-Pro-Gly-Glu-Asp (see, Table 2). This heterogenecity did not disappear by cloning, revealing that one cell produced a mixture of products having the different N-terminus.

Reference Example 2. Replacement of signal peptide (joined through Ala)

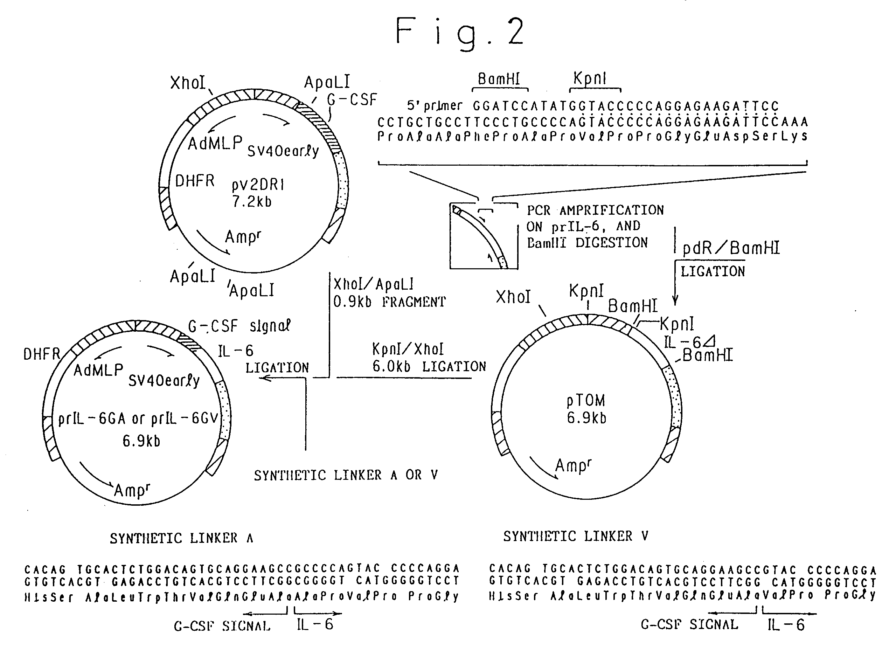

A gene sequence coding for a portion near the N-terminus of human IL-6 was mutated by a polymerase chain reaction method to introduce a new KpnI site (see, Fig. 2).

Namely, using a synthetic primer having the following sequence (GGATCCATATGGTACCCCCAGGAGAAGATTCC, CGTCGACGGATCCGGTGCCCATGCTAC) and as a template the plasmid prIL-6 containing a human IL-6 gene described in Reference Example 1, and using a Gene Amp kit and Thermal Cycler (Takara Shuzo), the fragment incorporating the mutation was amplified.

This fragment was treated with BamHI, and subcloned into the BamHI site of plasmid pdR as described in Reference Example 1 to construct plasmid pTOM (see, Fig. 2).

Linker A consisted of two synthetic oligonucleotides having the following sequences (TGCACTCTGGACAGTGCAGGAAGCCGCCCCAGTAC, and TGGGGCGGCTTCCTGCACTGTCCAGAG).

A KpnI/XhoI fragment (6.0 kb) of plasmid pTOM, an XhoI/ApaLI fragment (0.9 kb) of plasmid pV2DR1 containing human G-CSF gene (described in Japanese Examined Patent Publication (Kokoku) No. 1-5395; having the structure substantially identical with prIL-6) and the above-mentioned synthetic linker A were ligated to obtain plasmid prIL-6GA (see, Fig. 2). This plasmid encodes a signal peptide of human G-CSF linked to human IL-6 starting with Ala-Pro-Val-Pro-Pro-Gly-.

An analysis was carried out according to the same procedure as described in Reference Example 1 except that prIL-6GA was used in place of the plasmid prIL-6. As a result, again, the N-terminal amino acid sequences were determined as Ala-Pro-Val-Pro-Pro-Gly- and Val-Pro-Pro-Gly-Glu-Asp-.

Example 1. Replacement of signal peptide (joined through Val)

Linker V consisted of two oligonucleotides having the following sequences (TGCACTCTGGACAGTGCAGGAAGCCGTAC, and GGCTTCCTGCACTGTCCAGAG).

Plasmid prIL-6GV was obtained according to the same procedure as described in Reference Example 2 except that the above-mentioned synthetic linker V was used (see Fig. 2). This plasmid encodes a signal peptide of human G-CSF linked to human IL-6 starting with Val-Pro-Pro-Gly-Glu-Asp-.

An analysis was carried out according to the same procedure as described in Reference Example 1 except that prIL-6GV was used in place of plasmid prIL-6. As a result, the N-terminal amino acid sequence was homogeneous; Val-Pro-Pro-Gly-Glu-Asp- (see Table 2). In addition the differences in modification by sugar chains and biological activities were not found, and artificial modification did not have any effect thereon.

The result is summarized in the following Table 2.

Table 2

| Amino acid sequence of a portion near to N-terminus of purified recombinant human IL-6 the number in ( ) is pmol | | | | | | |
|---|---|---|---|---|---|---|
| Cycles | 1 | 2 | 3 | 4 | 5 | 6 |
| Reference Example 1 | Ala(203) Val( 45) | Pro(164) | Val(98) Pro(60) | Pro(127) Gly( 40) | Pro(138) Glu( 32) | Gly(95) Asp(37) |
| Reference Example 2 | Ala( 29) Val( 7) | Pro( 21) | Val(20) Pro( 7) | Pro( 20) Gly( 7) | Pro( 22) Glu( 3) | Gly(95) Asp( 4) |
| Example 1 | Val( 64) | Pro( 55) | Pro(56) | Gly( 50) | Glu( 28) | Asp(18) |

Example 2. Analysis of sugar chain structure of recombinant human IL-6 having Val at its N-terminus

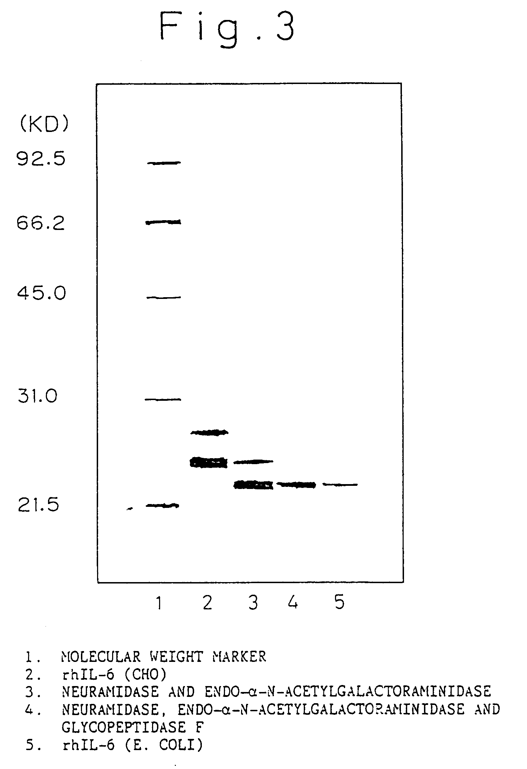

Where recombinant human IL-6 having Val at its N-terminus, obtained in Example 1 was subjected to SDS-polyacrylamide gel electrophoresis (abbreviated SDS-PAGE hereinafter) and Western blotting using an anti-rhIL-6 antibody, bands were detected at positions corresponding to molecular weights 24 kD and 28 kD (Fig. 3, lane 2), and it was found that the present IL-6 has a molecular weight higher than that of a recombinant human IL-6 from E. coli (Amerscham) (Fig. 3, lane 5; 22 kD). It is considered that the reason for the difference in molecular weight is because the present recombinant human IL-6 has sugar chains as in native human IL-6 (Van Dammc J. et al., J. Immunol., 140, 1534 (1988)).

Accordingly, to analyze the bonded sugar chains, the recombinant human IL-6 was digested with Neuramidase (Seikagaku Kogyo), endo-$\alpha$-N-acetylgalactosaminidase (Boehringer Mannheim) and glycopeptidase F (Boehringer Mannheim), and the change in molecular weight caused by the digestion was tested by SDS-PAGE and Western blotting. As a result, as can be seen in Fig. 3, the digestion with Neuramidase and end-$\alpha$-N-acetylglucosaminidase lowered the molecular weight as shown in lane 3, and by further digestion with glycopeptidase-F, a single band corresponding to a molecular weight of 22 kD which is the same as the molecular weight of recombinant human IL-6 derived from E. coli was detected as shown in lane 4.

The above-mentioned results can be summarized as follow: (1) considering substrate specificity of endo-$\alpha$-N-acetylgalactosaminidase (which cuts off Gal$\beta$1-3GalNAc from Ser or Thr), the recombinant human IL-6 has sugar chains wherein sialic acids(?) are bounded to the core of Gal$\beta$1-3GalNAc, similarly to native human IL-6. (2) some of molecules have N-linked type sugar chains similarly to native IL-6.

Accordingly to the present invention, homogeneous recombinant human IL-6 having Val at the N-terminus and modified with sugar chains is provided.

SEQUENCE LISTING

(1) GENERAL INFORMATION:

    (i) APPLICANT:
        (A) NAME: CHUGAI SEIYAKU KABUSHIKI KAISHA et al
        (B) STREET: 5-1, UKIMA 5-CHOME
        (C) CITY: TOKYO
        (E) COUNTRY: JAPAN
        (F) POSTAL CODE (ZIP): 115

    (ii) TITLE OF INVENTION: RECOMBINANT HUMAN INTERLEUKIN 6 WITH
        HOMOGENEOUS N-TERMINUS AND PRODUCTION THEREOF

    (iii) NUMBER OF SEQUENCES: 6

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25 (EPO)

(2) INFORMATION FOR SEQ ID NO: 1:

    (i) SEQUENCE CHARACTERISTICS:
        (A) LENGTH: 32 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO: 1:

GGATCCATAT GGTACCCCCA GGAGAAGATT CC                32

(2) INFORMATION FOR SEQ ID NO: 2:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO: 2:

CGTCGACGGA TCCGGTGCCC ATGCTAC                27

(2) INFORMATION FOR SEQ ID NO: 3:

    (i) SEQUENCE CHARACTERISTICS:
        (A) LENGTH: 35 base pairs

(B) TYPE: nucleic acid
(C) STRANDEDNESS: single
(D) TOPOLOGY: linear

(ii) MOLECULE TYPE: DNA (genomic)


(xi) SEQUENCE DESCRIPTION: SEQ ID NO: 3:

TGCACTCTGG ACAGTGCAGG AAGCCGCCCC AGTAC                    35

(2) INFORMATION FOR SEQ ID NO: 4:

        (i) SEQUENCE CHARACTERISTICS:
            (A) LENGTH: 27 base pairs
            (B) TYPE: nucleic acid
            (C) STRANDEDNESS: single
            (D) TOPOLOGY: linear

        (ii) MOLECULE TYPE: DNA (genomic)



(xi) SEQUENCE DESCRIPTION: SEQ ID NO: 4:

TGGGGCGGCT TCCTGCACTG TCCAGAG                             27

(2) INFORMATION FOR SEQ ID NO: 5:

        (i) SEQUENCE CHARACTERISTICS:
            (A) LENGTH: 29 base pairs
            (B) TYPE: nucleic acid
            (C) STRANDEDNESS: single
            (D) TOPOLOGY: linear

        (ii) MOLECULE TYPE: DNA (genomic)



(xi) SEQUENCE DESCRIPTION: SEQ ID NO: 5:

TGCACTCTGG ACAGTGCAGG AAGCCGTAC                           29

(2) INFORMATION FOR SEQ ID NO: 6:

        (i) SEQUENCE CHARACTERISTICS:
            (A) LENGTH: 21 base pairs
            (B) TYPE: nucleic acid
            (C) STRANDEDNESS: single
            (D) TOPOLOGY: linear

        (ii) MOLECULE TYPE: DNA (genomic)



(xi) SEQUENCE DESCRIPTION: SEQ ID NO: 6:

GGCTTCCTGC ACGTTCCAGA G                                   21


## Claims

1. Recombinant human interleukin-6 having a homogeneous N-terminus, comprising peptide molecular species whose N-terminus is Val and having sugar chains.

2. A process for production of recombinant human Interleukin-6 as defined in claim 1, characterized by culturing animal cells transformed with a DNA coding for a fusion protein comprising human interleukin-6 whose N-terminus is Val and a signal peptide other than the native signal peptide of human interleukin-6, and recovering the interleukin-6 from the resulting culture.

3. A process according to claim 2, wherein the signal peptide is a signal peptide of human granulocyte colony stimulation factor.

Fig.1

Fig. 2

SYNTHETIC LINKER A

```
CACAG TGCACTCTGGACAGTGCAGGAAGCCGCCCCAGTAC CCCCAGGA
GTGTCACGT GAGACCTGTCACGTCCTTCGGCGGGGT CATGGGGGTCCT
HisSer AlaLeuTrpThrValGlnGluAlaAlaProValPro ProGly
```

SYNTHETIC LINKER V

```
CACAG TGCACTCTGGACAGTGCAGGAAGCCGTAC CCCCAGGA
GTGTCACGT GAGACCTGTCACGTCCTTCGG CATGGGGGTCCT
HisSer AlaLeuTrpThrValGlnGluAlaValPro ProGly
```

# Fig.3

```
(KD)
92.5  ——
66.2  ——
45.0  ——
31.0  ——
21.5  ——
       1    2    3    4    5
```

1. MOLECULAR WEIGHT MARKER
2. rhIL-6 (CHO)
3. NEURAMIDASE AND ENDO-α-N-ACETYLGALACTORAMINIDASE
4. NEURAMIDASE, ENDO-α-N-ACETYLGALACTORAMINIDASE AND
   GLYCOPEPTIDASE F
5. rhIL-6 (E. COLI)

# INTERNATIONAL SEARCH REPORT

International Application No  PCT/JP92/00849

## I. CLASSIFICATION OF SUBJECT MATTER (if several classification symbols apply, indicate all) [6]

According to International Patent Classification (IPC) or to both National Classification and IPC

Int. $Cl^5$  C07K15/14, C12P21/02//C12N15/24 (C12P21/02,C12R1:91)

## II. FIELDS SEARCHED

| Minimum Documentation Searched [7] | |
|---|---|
| Classification System | Classification Symbols |
| IPC | C07K15/04, 15/14, C12P21/02, C12N15/19, 15/22, 15/24 |

Documentation Searched other than Minimum Documentation
to the Extent that such Documents are Included in the Fields Searched [8]

Biological Abstracts Data Base (BIOSIS)

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| X/A | European Journal of Biochemistry, Vol. 168, No. 3, (1987), J. Van Damme et al. "Purification and characterization of human fibroblast - derived hybridoma growth factor identical to T-cell-derived B-cell stimulatory factor-2 (interleukin-6)"  p. 543-550 | 1/2, 3 |
| A | Agricultural and Biological Chemistry, Vol. 54, No. 10, (1990), N. Tonouchi et al. "Hyper-heterogeneity of the N-terminus of recombinant BSF-2/IL-6 produced in CHO and NIH3T3 cells"  p. 2685-2688 | 1-3 |

* Special categories of cited documents: [10]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

## IV. CERTIFICATION

| Date of the Actual Completion of the International Search | Date of Mailing of this International Search Report |
|---|---|
| September 21, 1992 (21. 09. 92) | October 13, 1992 (13. 10. 92) |

| International Searching Authority | Signature of Authorized Officer |
|---|---|
| Japanese Patent Office | |

Form PCT/ISA/210 (second sheet) (January 1985)